# EUROPEAN PATENT APPLICATION

(11) **EP 4 826 380 A1**
(43) Date of publication of application: **21.10.2026**
(21) Application number: 25472015.4
(22) Date of filing: 16.04.2025
(51) Int. Cl.: G01N 33/28, G01N 21/41, G01N 21/64, G01N 21/85

(54) **ANTIFREEZE DETECTION SYSTEM IN ENGINE OIL**

(71) Applicant: Eood, Amissa Carly, 1000 Sofia (BG)
(72) Inventor: Aleksandrova, Monika, 1000 Sofia (BG)
(74) Representative: Nikolov, Vladislav Zdravkov

(57) **Abstract**

The invention provides a new and innovative system that combines various sensor technologies and is easy to use by both ordinary users and professional car repair shops. The system offers the possibility of IoT connectivity and integration with diagnostic platforms, making it useful for both individual users and car repair shops and diagnosticians.

## Description

### Field of the Invention

The invention relates to a system for detecting antifreeze in motor oil using combined sensor technologies to measure various chemical and physical parameters of the oil, including infrared spectroscopy (IR), refraction, electrical conductivity and fluorescence.

### Prior Art

The existing state of the art has various methods for detecting antifreeze in motor oils, including chemical tests and laboratory analysis. The use of sensors such as IR spectroscopy, refractometers and electrical conductivity sensors for motor oil analysis is known, but there is no compact, easy-to-use device that combines these technologies in a single mobile device with IoT connectivity and integration with diagnostic systems in car repair shops.

### Summary of the invention

The task of the invention is to provide a system for detecting antifreeze in engine oil, which combines various sensor technologies and data processing via a microcontroller, providing an easy-to-use, mobile and IoT-connected tool that can be used by both consumers and professional car repair shops.

This problem is solved by creating a system for detecting antifreeze in engine oil, including:
- a sampling system that includes a small pump or capillary tube for collecting the sample, connected to a filter for separating solid impurities from the sample,
- at least one analysis sensor selected from the group consisting of an IR spectrometer for detecting glycols, a refractometer for measuring light refraction, an electrical conductivity sensor for measuring conductivity and a UV-LED photodetector for measuring fluorescence,
- a microcontroller for processing collected data, connected to the ability to receive data in real time from at least one analysis sensor, and with the ability to send data to a visualization device or a logical unit of a cloud system,
- a microcontroller for controlling each of the sensors, configured to send commands to them, and to receive commands from a user interface with an information input device,
- a power source connected directly or indirectly to the sensors and microcontrollers.

Preferably, the system includes a logic block for IoT connectivity, with the microcontroller connected via a Wi-Fi connection or a mobile network to the cloud system.

Preferably, the microcontroller for processing the collected data is configured to create reports, detect trends in the condition of the engine oil and provide guidance for optimizing the engine diagnostic process based on previously entered reference information.

Preferably, the system includes a communication block for connecting to an automated diagnostic system in a car repair shop, with the sensors and the microcontroller being connected to diagnostic platforms in the repair shops with the ability to exchange data

Preferably, the system includes laser or chemical analysis devices connected to the microcontroller for processing the collected data.

Preferably, all sensors and microcontrollers of the system are combined in a single mobile device.

The invention provides a new and innovative system that combines different sensor technologies and is easy to use by both ordinary users and professional car repair shops.

The system offers the possibility of IoT connectivity and integration with diagnostic platforms, making it useful for both individual users and car repair shops and diagnosticians.

### Exemplary embodiment of the invention

The system for detecting antifreeze in engine oil according to the invention includes:
- A sampling system that includes a small pump or capillary tube for collecting the sample, connected to a filter for separating solid impurities from the sample;
- Analysis sensors selected from the group consisting of an IR spectrometer for detecting glycols, a refractometer for measuring the refraction of light in the oil, an electrical conductivity sensor for measuring conductivity and a UV-LED photodetector for measuring fluorescence;
- A microcontroller for processing the collected data, connected to the ability to receive data in real time from at least one sensor for analysis and to send data to a visualization device or a cloud system;
- A power source connected directly or indirectly to the sensors and microcontrollers.

The system for detecting antifreeze in engine oil includes combined technologies for measuring physical and chemical parameters of the oil.

The collected sample is filtered and analyzed using various sensors that can measure infrared radiation (IR spectroscopy), light refraction (refractometer), electrical conductivity and fluorescence.

The microcontroller processes the data from the sensors and allows visualization of the results in real time via an LCD display or a mobile application.

The system is equipped with IoT connectivity, which allows sending data to cloud platforms for further analysis or diagnostics.

The system can be integrated into a mobile device that combines all sensors and microcontrollers in one compact device.

The system can include a communication unit for connection to an automated diagnostic system in a car repair shop, which allows data exchange between the user device and the service platform.

Laser or chemical methods can be added for more accurate determination of the chemical components in the oil.

## Claims

1. A system for detecting antifreeze in engine oil, comprising:
• a sampling system that includes a small pump or capillary tube for collecting the sample, connected to a filter for separating solid impurities from the sample,
• at least one analysis sensor selected from the group consisting of an IR spectrometer for detecting glycols, a refractometer for measuring light refraction, an electrical conductivity sensor for measuring conductivity and a UV-LED photodetector for measuring fluorescence,
• a microcontroller for processing collected data, connected to the ability to receive data in real time from the at least one analysis sensor, and with the ability to send data to a visualization device or a logic unit of a cloud system,
• a microcontroller for controlling each of the sensors, configured to send commands to them, and with the ability to receive commands from a user interface with an information input device, • a power source connected directly or indirectly to the sensors and microcontrollers.

2. The system according to claim 1, comprising a logic unit for IoT connectivity, the microcontroller being connected via a Wi-Fi connection or a mobile network to the cloud system.

3. The system according to any of the preceding claims, wherein the microcontroller for processing the collected data is configured to generate reports, detect trends in the condition of the engine oil, and provide guidance for optimizing the engine diagnostic process based on previously entered reference information.

4. The system according to any of the preceding claims, comprising a communication unit for connecting to an automated diagnostic system in a car repair shop, the sensors and the microcontroller being connected to diagnostic platforms in the repair shops with the ability to exchange data

5. The system according to any of the preceding claims, comprising laser or chemical analysis devices connected to the microcontroller for processing the collected data.

6. A system according to any of the preceding claims, **characterized in that** all sensors and microcontrollers of the system are combined in one mobile device.
